# EUROPEAN PATENT APPLICATION

(11) **EP 4 119 651 A1**
(43) Date of publication of application: **18.01.2023**
(21) Application number: 21186246.1
(22) Date of filing: 16.07.2021
(51) Int. Cl.: C12M 1/12, C12M 3/00, C12M 1/06, C12M 1/00, B01F 33/00

(54) **CONSUMABLE FOR A BIOREACTOR SYSTEM**

(71) Applicant: The Automation Partnership (Cambridge) Ltd., Hertfordshire SG8 5WY (GB)
(72) Inventor: VARLEY, Mark, Hardwick, CB23 7XG (GB); SULLIVAN, Sean, SG7 6TW, Baldock (GB)
(74) Representative: Mewburn Ellis LLP

(57) **Abstract**

A consumable (1) for a bioreactor system is provided. The consumable comprises a plurality of bioreaction vessels (2), each suitable for holding the contents of a bioreaction. The plurality of bioreaction vessels are provided as a single-piece construction, and each bioreaction vessel includes an agitator (4) configured to agitate the contents of the respective bioreaction vessel. Each agitator has a stirring end which extends into the bioreaction vessel and a drive end.

## Description

### Field of the Invention

The present invention relates to a bioreactor system consumable comprising a plurality of bioreaction vessels.

### Background

Bioreactor systems are used to closely control the environments of cell culture solutions. In particular, the mixing of liquid and cells is critical to improving homogeneity within a bioreactor and hence control of the local environment to which cells within the bioreactor are exposed. To improve the ratio of success and speed to market of new biopharmaceutical products, there is a need to screen larger numbers of potential cell clones in small-scale production-like processes using bioreactor systems. This ensures the most suitable clones can be successfully identified and taken forward to a larger-scale development process.

Bioreactor systems with a plurality of small-scale bioreactors can be used to mimic larger scale cell culture environments. Ideally, when studying clone properties, cell culture conditions are uniform across the plurality of bioreactors to ensure that any differences observed in the produced cell clones can be attributed to the properties of the clones and not to individual bioreactors. For instance, stirring speed, temperature, DO (dissolved oxygen), pH set-point, allowable gas flows, feeding strategy and glucose set-point should be identical from bioreactor to bioreactor. The cell culture conditions should also be uniform within each individual bioreactor.

EP 2270129 A proposes a microscale bioreactor system comprising multiple single-use bioreaction vessels for containing cell cultures. An impeller-style agitator is provided for every bioreaction vessel to stir the contents of the vessel. At the beginning of an experiment each bioreaction vessel is individually loaded into the system by an operator. Furthermore, each agitator is manually orientated and connected to an external drive mechanism. This can be laborious and time consuming. More generally, the small size of the components and the large numbers of small parts mean that such microscale bioreactor systems are challenging to assemble and manufacture, leading to increased scrappage due to assembly errors.

Therefore, a bioreactor system is desired which is easier to assemble, efficient to load and unload between experiment runs, and provides uniform and controlled culture conditions.

### Summary of the Invention

Accordingly, in general terms the present invention provides a consumable for a bioreactor system, the consumable comprising:
a plurality of bioreaction vessels, each suitable for holding the contents of a bioreaction,
wherein each bioreaction vessel includes an agitator configured to agitate the contents of the respective bioreaction vessel, each agitator having a stirring end which extends into the bioreaction vessel and a drive end.

In a first aspect, the present invention provides a consumable for a bioreactor system, the consumable comprising:
a plurality of bioreaction vessels, each suitable for holding the contents of a bioreaction,
wherein the plurality of bioreaction vessels are provided as a single-piece construction, and each bioreaction vessel includes an agitator configured to agitate the contents of the respective bioreaction vessel, each agitator having a stirring end which extends into the bioreaction vessel and a drive end.

Advantageously, such a system reduces the assembly time involved to set-up a bioreactor system and improves the efficiency of the turn-around between experiment runs.

Optional features of the invention will now be set out.

The consumable may further comprise a drive mechanism connected or connectable to the drive ends of the agitators of the plurality of bioreaction vessels and configured to move the agitators with identical synchronised motions. The identical synchronised motions are preferably rotary synchronised motions, but can be other motions, such as linear reciprocating synchronised motions. Such an arrangement reduces the number of connections to be made by an operator when setting up a bioreaction system and avoids the need to manually position each agitator. The consumable may be provided to a user as a single unit (i.e. with the drive mechanism already connected to the drive end) which may be loaded into a bioreaction system. Alternatively, the consumable may be provided to a user as a two-part kit wherein one part comprises the bioreaction vessels and agitators, and the other part comprises the drive mechanism which may only be connected to the agitators upon loading to the bioreaction system.

The plurality of bioreaction vessels may be arranged in one or more rows and the drive mechanism may be a stirrer bar extending adjacent to and parallel with the one or more rows. Such an arrangement helps to ensure that each agitator receives the same driving motion, and is simpler to manufacture and has fewer moving parts than individual, dedicated drive mechanisms driving each vessel. In addition, having the vessels arranged in rows is compatible with a modular system in which one or more further consumables may be mounted adjacent a first consumable, all with parallel rows and stirrer bars, to increase in an orderly fashion the number of available vessels.

Indeed, more generally, in a second aspect, the present invention provides a consumable for a bioreactor system, the consumable comprising:
a plurality of bioreaction vessels arranged in one or more rows, each vessel being suitable for holding the contents of a bioreaction,
wherein each bioreaction vessel includes an agitator configured to agitate the contents of the respective bioreaction vessel, each agitator having a stirring end which extends into the bioreaction vessel and a drive end; and
wherein the consumable further comprises a stirrer bar which extends adjacent to and parallel with the one or more rows, which is connected or connectable to the drive ends of the agitators of the plurality of bioreaction vessels, and which is configured to move the agitators with identical synchronised motions.

Further optional features of the invention will now be set out. These are applicable singly or in any combination with any aspect of the invention.

Typically the stirring end of each agitator extends downwards into the bioreaction vessel from its drive end.

The drive mechanism may include an interface to an external drive linkage which allows connection to an external source of motive power provided in the bioreactor system. This facilitates integration of the consumable with the system.

The stirring ends of the agitators may be paddles. Such an arrangement is compatible with an orbital stirring motion of the paddles, which can reduce or avoid relative sliding and/or rotary motion between parts in the bioreaction vessels. This in turn results in less wear at the parts. Reducing the wear of parts in the bioreaction vessels reduces potential contamination of the bioreactions by micro-particles released by the wearing parts. Moreover, reduced part wear ensures the agitators are less likely to break and the agitation effects produced by the agitators are uniform from the beginning of an experiment to the end of the experiment. Top-mounted paddles in particular can avoid having parts where there is relative sliding and/or rotary motion within the contents of the bioreaction, thereby avoiding cell damage from such motion. Furthermore, agitators which are paddles present a similar agitation surface to the bioreaction fluid at all depths, irrespective of how far the paddle is submerged in the fluid. Therefore, the paddle agitators can accommodate a larger range of fluid volumes in the bioreaction vessel without causing unwanted foam effects or differing surface agitator properties even when the bioreaction vessel is only partially full. In contrast, impeller agitators should be fully submerged to reduce foaming and cell damage.

Conveniently, the paddle agitators may be connected to the drive mechanism at their drive ends via respective ball and socket joints, or via respective living hinges. Both types of joint can allow orbital motion (a form of rotary motion) at the drive ends which provides corresponding orbital motion of the paddles. Living hinges allow the agitators and drive mechanism to be formed as one part (e.g. a single injection moulding), reducing the number of manufacturing steps.

The paddles may be tapered such that the widths of the paddles decrease with increasing distance from their drive ends. Such a tapered paddle design can prevent the distal ends of the paddles from touching the side walls of the bioreaction vessels where the radial displacement of the paddles is at a maximum during orbital motion. Therefore, the end of paddle can extend closer to the bottom of the bioreaction vessel, reducing the minimum volume of bioreaction fluid which is required in the vessel. Alternatively or additionally, the paddles may comprise holes, slots and/or additional paddle surfaces to improve fluid mixing in the bioreaction vessels.

Each agitator typically has a pivot point for its synchronised motion between its drive end and its paddle. Each bioreaction vessel may further include a flexible element around the respective agitator at the pivot point, the flexible element providing a seal which closes off the bioreaction vessel at the agitator. Thus, the flexible element can help to prevent off-gas or fluid from the bioreaction escaping the bioreaction vessel at the entry point of the agitator to the bioreaction vessel, promoting a controlled environment inside the vessel. Conveniently, it does this while being located at a position along the agitator where the amplitude of the agitator's motion is at a minimum, helping to reduce the amount of relative motion between parts. This in turn results in less wear at the parts, thereby reducing contamination of the bioreaction. The flexible element may be made of a thermoplastic elastomer or silicone, or any other suitable elastomeric material that is biocompatible, able to form a seal, and can be sterilised by irradiation, autoclaving, EtO (Ethylene Oxide) or using a similar method.

The flexible element may be fastened to the bioreaction vessel, for example using a separate retaining element which clamps the flexible element in place. The flexible element may be keyed to the bioreaction vessel to prevent rotation of the flexible element relative to the vessel. Conveniently, the flexible element may be over-moulded around the agitator.

As an alternative to paddles, the stirring ends of the agitators may be impellers. In this case the identical synchronised motions of the agitators are rotary synchronised motions which turn the impellers about respective axes thereof. The agitators may have one or more blades on their stirring ends and may be configured to rotate in the vessel such that the contents of the bioreaction vessel are stirred by the blades. Particularly when the stirring ends of the agitators are impellers, it may be convenient for the consumable to be provided to a user as a two-part kit wherein one part comprises the bioreaction vessels and agitators, and the other part comprises the drive mechanism which is then connectable to the drive end of each agitator.

The bioreaction vessels may comprise respective outgassing ports for exhausting gas from the bioreaction. Each outgassing port may include a tortuous gas path for controlled gas exhaust therethrough. For example, tortuous gas path can help a slight positive pressure to be maintained in the vessels relative to the outside, whereby the expulsion rate and mixing of exhaust gases can be controlled, and ingress of external air by back flow along the path is discouraged. Moreover, the tortuous gas path can ensure that any incoming gases mix with existing gases in a headspace formed between the top surface of the bioreaction and a lid of the vessel instead of directly exiting through the outgassing port before this mixing can occur.

The plurality of the bioreaction vessels may be rigid containers. Conveniently they can be formed of injection moulded or blow moulded plastic. Typically they are clear-walled to allow their contents to be viewed.

In a second aspect, the present invention provides a bioreactor system comprising the consumable of the first aspect and a source of motive power for moving the agitators of the consumable. The system may include a drive linkage which interfaces at one end to a drive mechanism of the consumable and which connects at another end to the source of motive power to transmit the motive power to the drive mechanism.

The bioreactor system may further comprise a control unit for programmably controlling the movement of the agitators.

The present invention includes combination of any of the aspects and optional features described, except where such a combination is clearly impermissible or expressly avoided.

### Summary of the Figures

Embodiments of the invention will now be described by way of example with reference to the accompanying drawings in which:
Figure 1 shows a perspective view of a first embodiment of a consumable for a bioreactor system;
Figures 2A to 2D show respectively a plan view, a side elevation view, a sectional view along plane A-A, and a sectional view along plane B-B of the consumable of Figure 1;
Figure 3A shows a sectioned perspective view of the consumable of Figure 1, and Figure 3B shows a detailed view of section region C of Figure 3A;
Figure 4A shows a variant of a stirrer bar of the consumable of Figure 1, and Figure 4B shows a detailed view of region D of Figure 4A;
Figures 5A and 5B show a perspective view and a plan view respectively of a paddle agitator providing a linear reciprocating motion;
Figures 6A and 6B show a perspective view and a plan view respectively of a paddle agitator providing an orbital motion;
Figure 7 shows variants of a paddle agitator for use in the consumable of Figure 1;
Figure 8A shows a further plan view of the consumable of Figure 1, and Figure 8B shows a detailed view of region E of Figure 8A;
Figure 9 shows a further side view of the consumable of Figure 1;
Figure 10 shows a perspective view of a second embodiment of a consumable for a bioreactor system, a drive mechanism of the consumable not being shown;
Figures 11A to 11D show respectively a plan view, a side elevation view, a sectional view along plane B-B, and a sectional view along plane C-C of the consumable of Figure 10, again a drive mechanism of the consumable not being shown;
Figure 12 shows a perspective view of part of the consumable of Figure 10 with impeller agitators removed from some bioreaction vessels, and with vessel lids, a handle and a drive mechanism of the consumable not being shown;
Figures 13A to 13C show respectively a plan view, an elevation view and a sectional view along plane F-F of a variant of two of the consumables of Figure 10 joined together in a modular fashion; and
Figure 14A shows a perspective transparent view of an impeller connector and Figure 14B shows a perspective view of an interfacing connector which connects a drive shaft to the impeller connector.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference

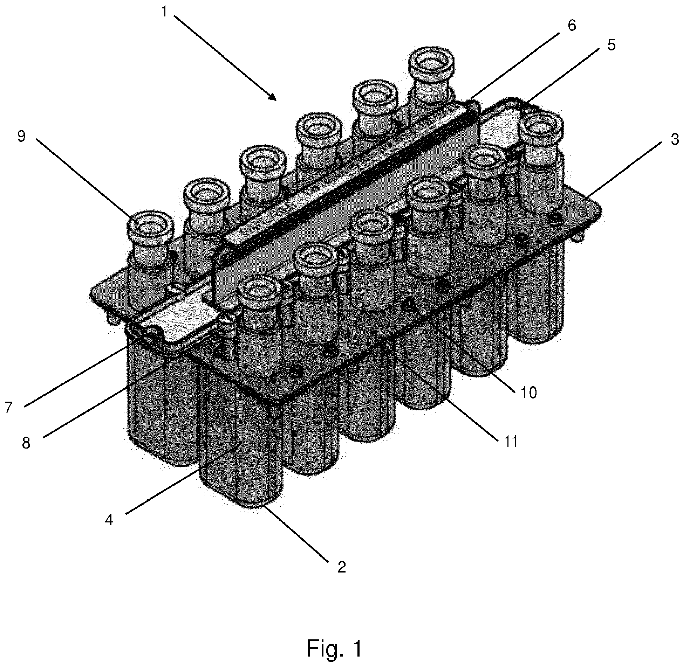

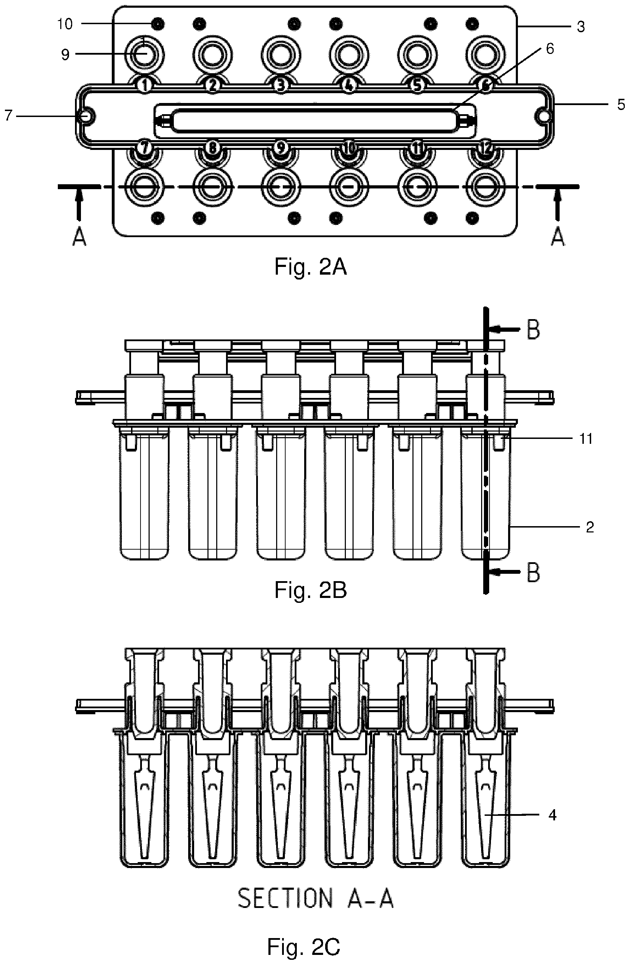

Figure 1 shows a perspective view of a first embodiment of a multi-parallel, single-use consumable for a bioreactor system, and Figures 2A to 2D show respectively a plan view, a side elevation view, a sectional view along plane A-A, and a sectional view along plane B-B of the consumable of Figure 1. The consumable 1 comprises a plurality of rigid bioreaction vessels 2 (two parallel rows are shown, each row having six vessels) suitable for holding the contents of a bioreaction. Each bioreaction vessel includes an agitator 4 for agitating the contents of the respective vessel, the agitator having a stirring end in the form of a paddle which extends into the bioreaction vessel and a drive end outside the vessel. The bioreaction vessels are a single piece construction, and conveniently may be formed of injection moulded or blow moulded plastic. The vessels are configured to be serviceable by a robotic liquid handler. Alternatively, they can be serviced manually.

The bioreaction vessels 2 are closed by a lid 3, with the agitators 4 extending through respective apertures formed in the lid. Typically, the lid is ultrasonically welded to the vessels. However, alternative arrangements may be possible. For example, each vessel or subset of vessels may have its own lid. In the embodiment shown, each vessel is configured to support a bioreaction working volume of between 0.5mL and 3.5mL and the consumable is anticipated to be in continuous use for bioreactions lasting typically 2 weeks but could extend up to 4 weeks. However, the vessels may be sized to support other working volumes, and the consumable adapted to last for shorter or longer durations.

The consumable 1 allows an operator to load the bioreaction vessels 2 in batches. Variants of the consumable may have any number of the vessels and/or different arrangements of the vessels within the consumable. For example, a consumable with 24 or 36 bioreaction vessels may be provided for larger scale experiments. Similarly, the 12 bioreaction vessels may be provided in the 2 by 6 arrangement shown in Figure 1, or in a 3 by 4 arrangement. The multiple vessels form a "rack" which can be loaded in one action, and which has a wide footprint that ensures the consumable is stable when standing on a bench.

A drive mechanism in the form of a stirrer bar 5 is connected to the drive ends of the agitators 4 by respective ball and socket joints 8. The stirrer bar extends between and parallel to the rows of vessels 2 so that each agitator receives the same driving motion. The stirrer bar has holes 7 at both ends which interface to an external drive linkage (not shown) for connecting to an external source of motive power of the bioreactor system, such as an electric motor. The linkage moves the stirrer bar back and forth or with a circulating motion which induces in the ball and socket joints respectively a linear reciprocating motion or an orbital motion. As discussed in more detail below, this motion of the joints then induces a corresponding motion of the paddles, thereby stirring the contents of the bioreaction. The paddles reduce the shear stress on the cells of the bioreaction and provide good stirring conditions across a wide range of working volumes.

The stirrer bar is 5 provided as part of the consumable 1 and links all of (or a subset of) the agitators 4 in the consumable together. Therefore, the induced motions of the paddles are identical synchronous motions. This ensures consistency in the bioreaction environments across the plurality of bioreaction vessels. Furthermore, such an arrangement is simpler to manufacture and has fewer moving parts than individual, dedicated drive mechanisms driving each agitator. A programmable control unit (not shown) controls the electric motor and linkage, and thereby controls the type, speed and duration of the paddle motion.

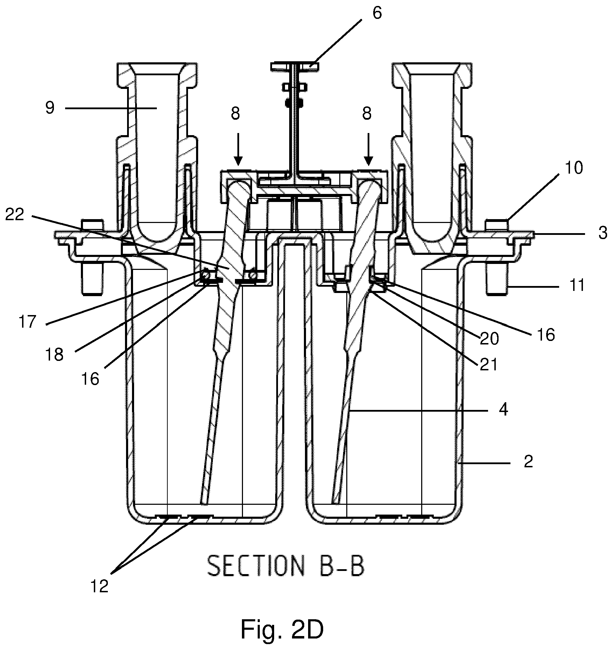

A handle 6 extends upwards from the lid 3 through a slot in the stirrer bar 5. Moreover, a flange protrudes from both sides of the handle above the slot in the stirrer bar to prevent the stirrer bar detaching from the consumable. Typically, a unique identifier such as a bar code is included on the handle to identify the batch of bioreactions contained in the bioreaction vessels 2. Each bioreaction vessel 2 includes a capped sample port 9 to allow fluid to be added to the vessel or samples to be taken from the vessel, e.g. by a pipette. Each sample port can have its own separate cap, as shown in Figure 1. Alternatively, groups of sample ports can share integrated caps, e.g. in the example of Figure 1 and 2 each row of six vessels 2 can have a six-way integrated cap that allows all the vessels of the row to be opened and closed by respectively removal and fitting of the single integrated cap. Sensors 12 are integrated in the base of each vessel to allow properties of the bioreactions to be monitored (for example, pH or DO).

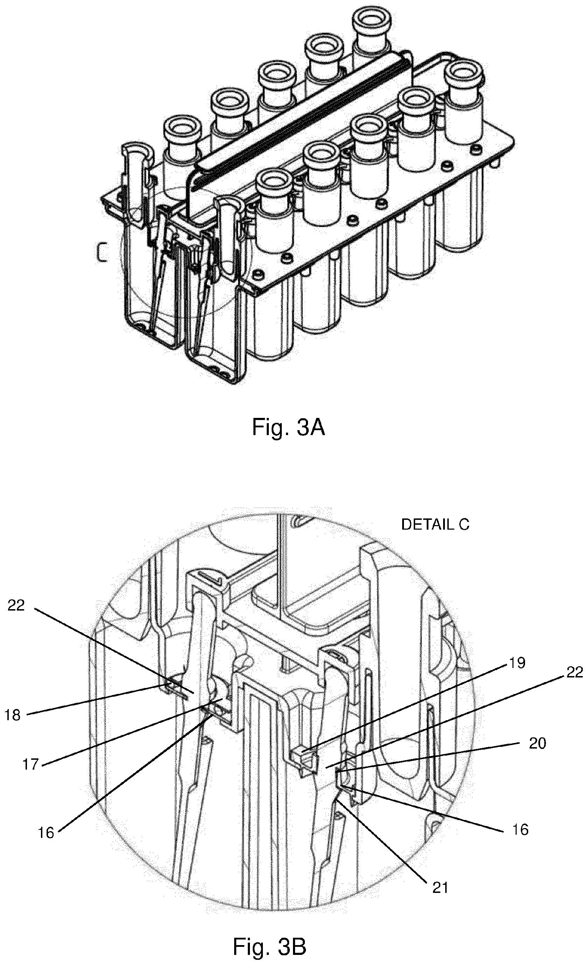

Figure 3A shows a sectioned perspective view of the consumable of Figure 1, and Figure 3B shows a detailed view of section region C of Figure 3A. Flexible elements 16 are provided where the agitators 4 extend through the respective apertures formed in the lid 3. Each flexible element provides a deformable, sterile seal between its agitator and the lid 3 that helps to prevent off-gases from the bioreaction escaping through the aperture in the lid. Thus, a controlled gas composition can be maintained in the headspace between the bioreaction fluid and the lid. The seal also helps to prevent contamination of the bioreaction from external agents. Moreover, the flexible element also helps to maintain the vertical position of the agitator in its vessel, and also constrains any sideways motion at the level of the flexible element. In this way, when the agitator is moved by the stirrer bar 5, the flexible element deforms slightly to accommodate the movement while maintaining the seal. The flexible element is able to do this because it effectively defines a pivot point 22 of the agitator between on one side the driven motion of the ball and socket joint and on the other side the corresponding induced motion of the paddle.

The flexible element 16 may be made of silicone or polyethylene or any other suitable elastomer material which is biocompatible and sterilisable. An index tab 19 is provided on the flexible element for keying to a respective slot formed the lid. This prevents the flexible element, and the agitator 4, from rotating about the centre of the respective aperture. Conveniently, the whole stirring mechanism can be sterilised e.g. using irradiation, autoclaving or EtO.

The sections of Figures 2D, 3A and most clearly 3B show two different arrangements to secure the agitators 4 and the flexible elements 16 to the lid 3. A first arrangement is shown in the left vessel wherein the flexible element 16 is a planar elastomeric annulus resting at its outer perimeter on a rim formed by the lid 3 around the aperture, while the inner perimeter of the annulus fits snugly in a circumferential groove formed in the agitator. An O-ring 18 is provided above the flexible element to perfect the seal, and is secured in place by a retaining element 17 (which also has an index tab to prevent its rotation about the agitator).

A second locking arrangement is shown in the right vessel 2 wherein the agitator 4 is held in place by just the flexible element 16. In this case, the circumferential groove 20 formed in the agitator is more substantial, and the inner perimeter of the flexible element is sized to match. Moreover, the outer circumference of the flexible element has a groove formed therein which receives the edge of the rim formed by the lid. This arrangement is more suitable for forming the flexible element 16 as an over-mould around the agitator 4.

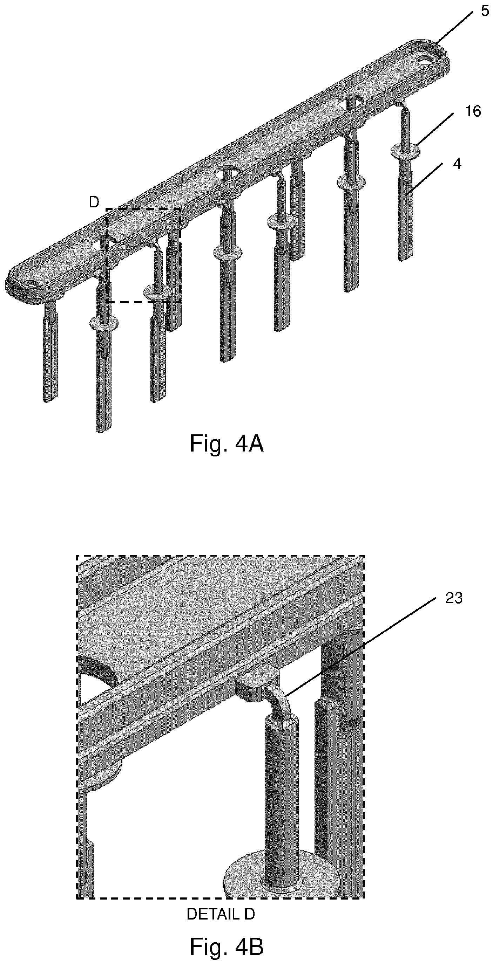

Figure 4A shows a variant of the stirrer bar 5 of the consumable of Figure 1, in which the agitators are connected to the stirrer bar using living hinges 23 instead of ball and socket joints. Figure 4B shows a detailed view of region D of Figure 4A. In this variant, the agitators 4 and stirrer bar 5 may be formed as one part, for example by injection moulding, reducing the number of manufacturing steps.

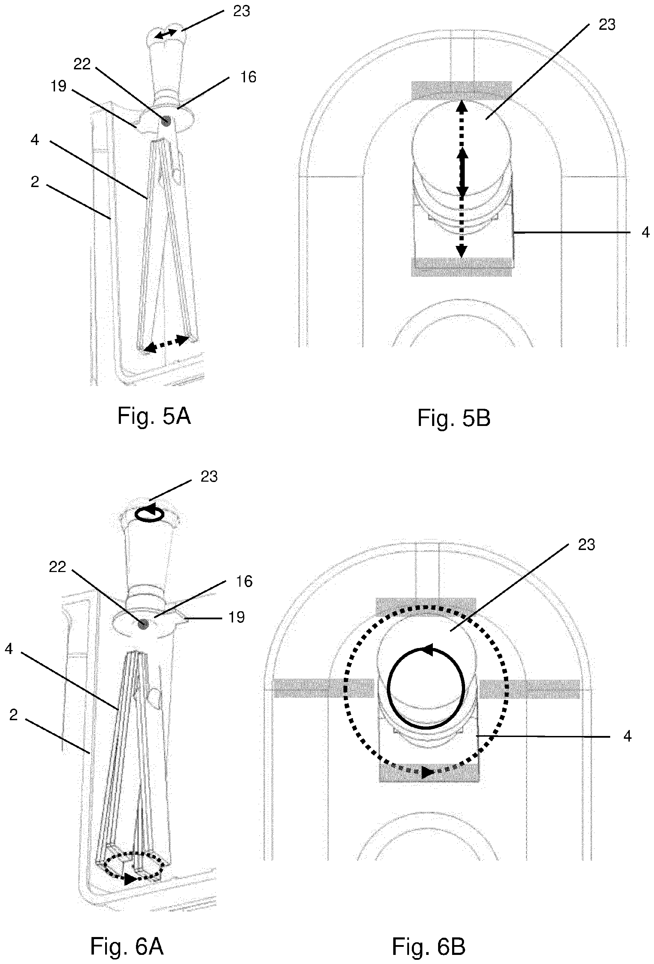

Figures 5A and 5B show a perspective view and a plan view respectively of a paddle agitator 4 undergoing a linear reciprocating motion. The drive end of the agitator, corresponding to the ball and socket joint 23, is driven via a back and forth movement of the stirrer bar 5 along a linear reciprocating path, as indicated by the solid arrowed lines. The agitator pivots about the pivot point 22 and the distal end of the paddle on the opposite side of the pivot point follows a corresponding linear reciprocating path, indicated by the dashed arrowed lines at the bottom of the bioreaction vessel 2. The ratio of the amplitudes of the reciprocating paths at the ball and socket joint 23 and the distal end of the paddle depends on the ratio of the distances of the ball and socket joint and the distal end of the paddle to the pivot point.

Figures 6A to 6B show a perspective view and a plan view respectively of a paddle agitator 4 undergoing an orbital motion. The drive end of the agitator, corresponding to the ball and socket joint 23 is driven via a circulating movement of the stirrer bar 5 along a circular orbit indicated by the solid arrowed line. The distal end of the paddle describes a corresponding circular orbit at the bottom of the bioreaction vessel 2, as indicated by the dotted arrowed line. The ratio of the diameters of the circular orbits at the ball and socket joint 23 and the distal end of the paddle depends on the ratio of the distances of the ball and socket joint and the distal end of the paddle to the pivot point.

The motion of the paddles 4 is not limited to circular orbital or linear reciprocating motions. For example, the paddles may be driven in figure-of-eight or elliptical motions.

A benefit of paddle agitators 4 is that the motion of the paddles results in agitation of both the bioreaction fluid and the head space gas above the bioreaction fluid. Moreover, the paddles can be formed as single pieces, which do not require assembly of multiple parts. Typically, they are made of rigid plastic. Unlike conventional stirred impellers, the paddles avoid having relative sliding and/or rotary motion between parts that are immersed in the bioreaction fluid. They also enable a securing arrangement that is less susceptible to wear, rubbing, grinding and fatigue damage than conventional stirred impellers, helping to avoid the creation of wear particulates within the vessels 2 and reducing vibration and noise.

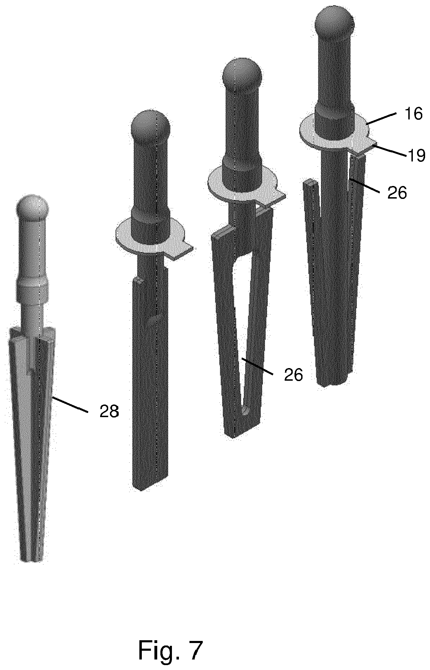

Figure 7 shows variant shapes of the paddle agitator 4. The paddles may have one or more of: holes or slots 26 formed in the paddle, additional paddle surfaces 28, and tapered or non-tapered profiles.

Different paddle geometries may be used to achieve a desired kLa (gas transfer) and shear profile for cells. As described above a tapered profile can accommodate a large range of fluid volumes in the vessel without causing unwanted foam affects or differing surface agitator properties. For example, cells in 0.5 mL of bioreaction fluid will see substantially the same forces as cells in 3.5 mL of fluid, as the paddle traverses the entire working volume. In contrast, with a conventional impeller, cells in different volumes of fluid can experience different conditions, and particularly if the fluid drops below the top level of the impeller to encourage foaming.

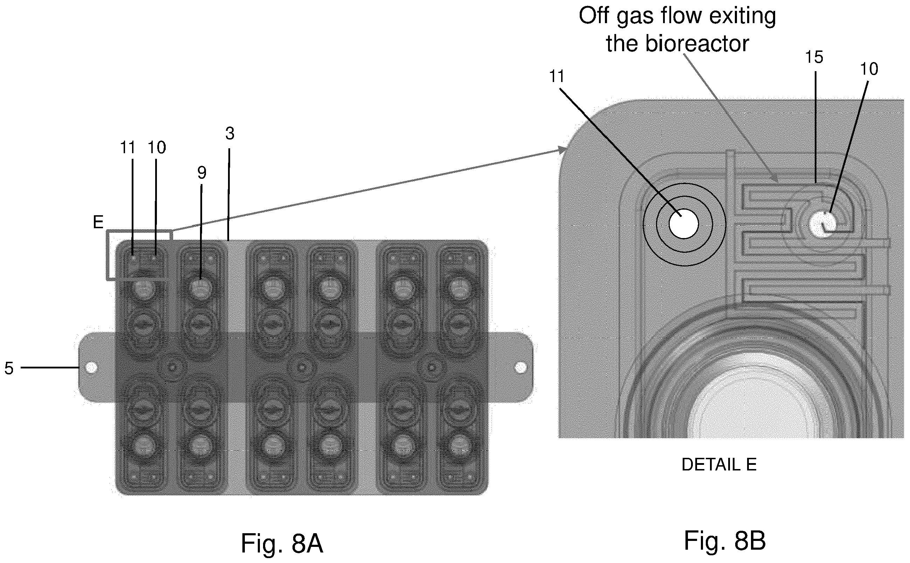

Figure 8A shows a further plan view of the consumable of Figure 1, and Figure 8B shows a detailed view of region E of Figure 8A including an exhaust channel 15. Each bioreaction vessel 2 includes such an exhaust channel to allow the exhaustion of gases (off-gases) which may be products of the bioreaction. Additionally, an inlet gas port 11 is provided for each vessel 2 for introducing gases to the vessel. Off-gas is expelled from the vessel into the exhaust channel 15 leading to an outlet gas port 10 in the lid 3. The exhaust channel follows a tortuous route to the outlet gas port. For instance, the exhaust channel may be long and narrow and have multiple bends. Such a tortuous route encourages a slight positive pressure in the vessel 2, reduces the rate of gas flowout of the vessel, reduces evaporation of the bioreaction fluid, and ensures that the off-gases and introduced gases are suitably mixed before being released. Furthermore, the exhaust channel allows the off-gases to be sensed as they exit the vessel. The exhaust channel may be inclined to allow the collection of any condensate back into the vessel 2. Alternatively or additionally, in some arrangements, the off-gases may be allowed to exhaust through gas escape routes provided by imperfect seals between the agitators 4 and the lid 3.

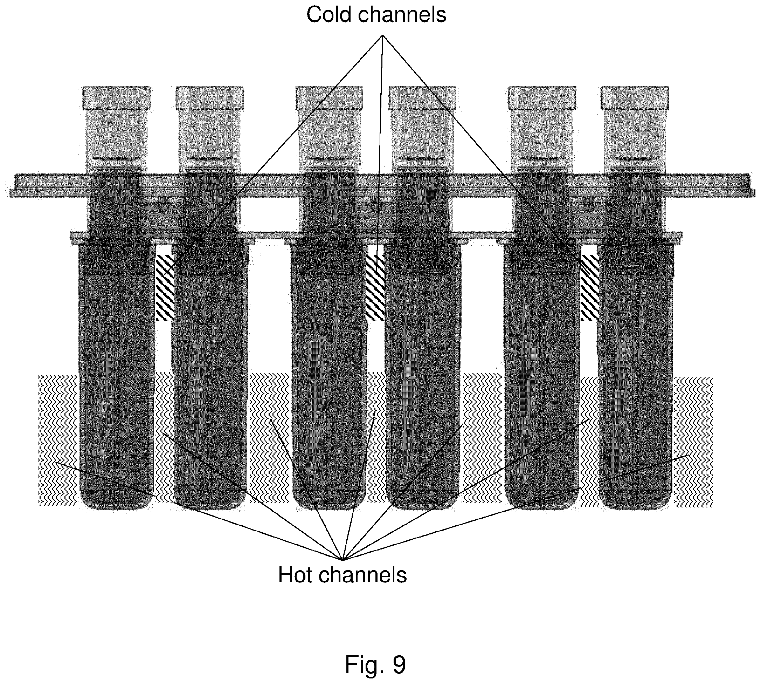

Figure 9 shows a further side view of the consumable of Figure 1, illustrating cold and hot channels. Cooling elements can be provided in the bioreaction system at the cold channels to promote condensation of off-gases in the exhaust channels 15 so that evaporation fluid losses are reduced. The bioreaction vessels 2 are arranged in mirrored pairs. This ensures that the exhaust channels of each vessel in a pair are arranged close to each other and share a cooling element. Thus cooling of the off-gases can be performed with fewer cooling elements. The hot channels meanwhile enable temperature-control of the contents of the bioreaction vessels by conduction of heat into the vessels from these channels, e.g. by loading the consumable into a solid state heating block having heated projections extending into the channels, or by providing heated airflows along the channels.

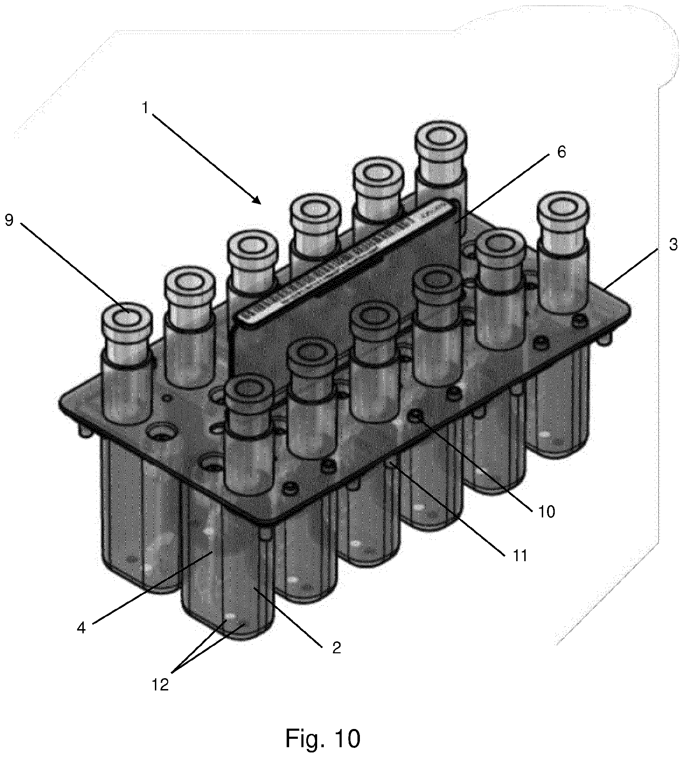

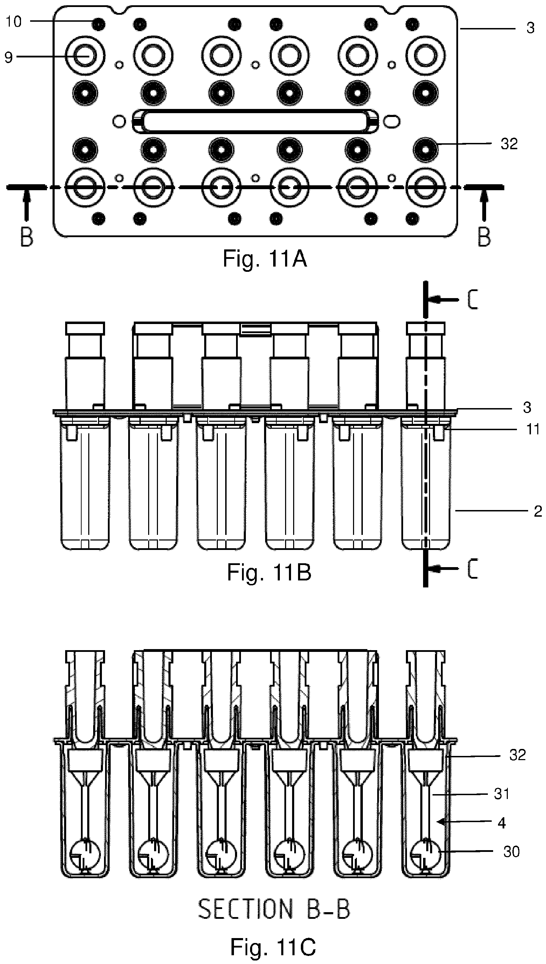

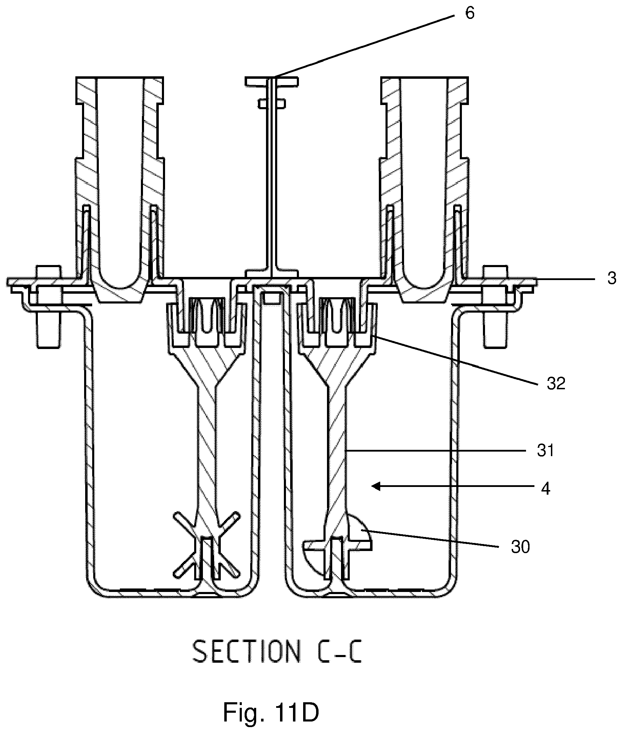

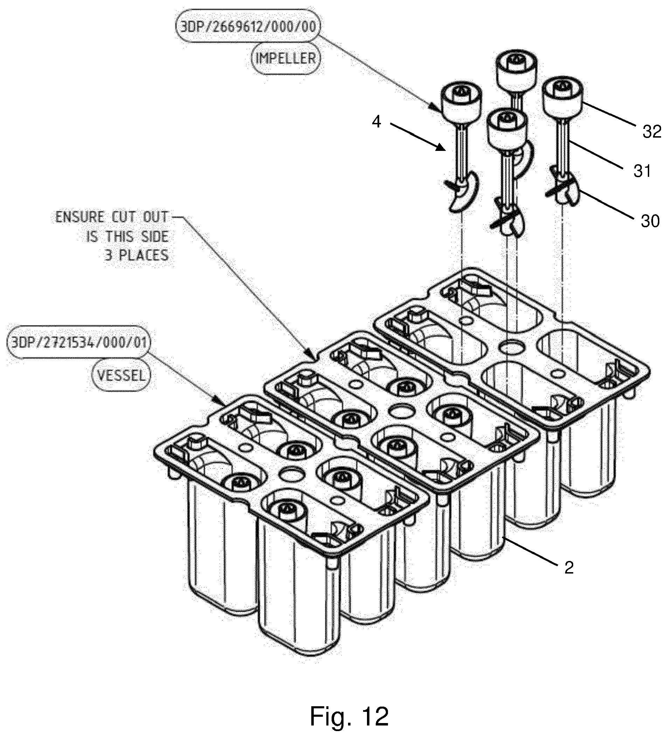

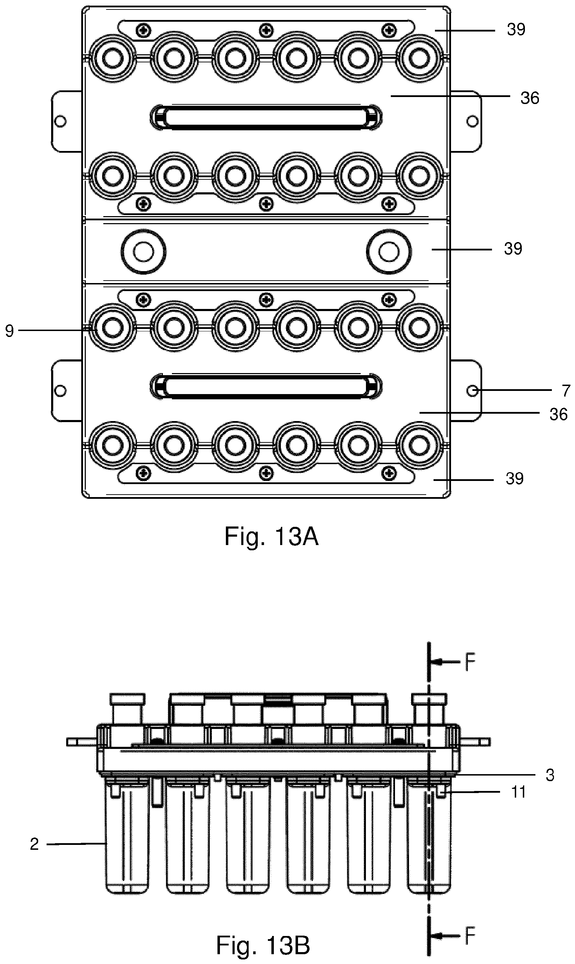

Figure 10 shows a perspective view of a second embodiment of a consumable for a bioreactor system, a drive mechanism of the consumable being omitted; Figures 11A to 11D show respectively a plan view, a side elevation view, a sectional view along plane B-B, and a sectional view along plane C-C of the consumable of Figure 10; and Figure 12 shows a perspective view of part of the consumable of Figure 10 with agitators 4 removed from some bioreaction vessels 2. Additionally, Figures 13A to 13C show respectively a plan view, an elevation view and a sectional view along plane F-F of two of the consumables of Figures 10 to 12 joined together in a modular fashion. The consumable comprises a plurality of bioreaction vessels 2 closed by respective lids 3, as in the consumable of the first embodiment. Side plates 39 hold the lid 3 in place at the sides of the consumable and allow adjacent consumables to be joined together, as shown in Figures 13A to 13C. However, the agitators 4 of the second embodiment are impellers, each impeller comprising a shaft 31 extending into its respective bioreaction vessel, and blades 30 at the bottom end of the shaft for stirring the contents of the bioreaction. The top end of each shaft terminates in an impeller connector 32 which fits loosely around sides of a respective well formed in the lid. In this way the impeller connector 32 forms a labyrinth with the sides of the well which can catch any wear particulates formed by the drive mechanism, helping to prevent contamination of the content of the bioreaction vessel.

The well has an aperture at its bottom through which a drive shaft 33 (described below) joins to the impeller connector 32. A further contrast with the consumable of the first embodiment is that rather than having all the bioreaction vessels 2 formed by one single piece construction, they are formed by several (three shown in Figure 12) single piece constructions, each construction providing plural (four shown in Figure 12) of the vessels. The single piece constructions are then located relative to each other by the lid 3.

In the embodiment shown, each vessel is configured to support a bioreaction working volume of between 1.5mL and 3.5mL.

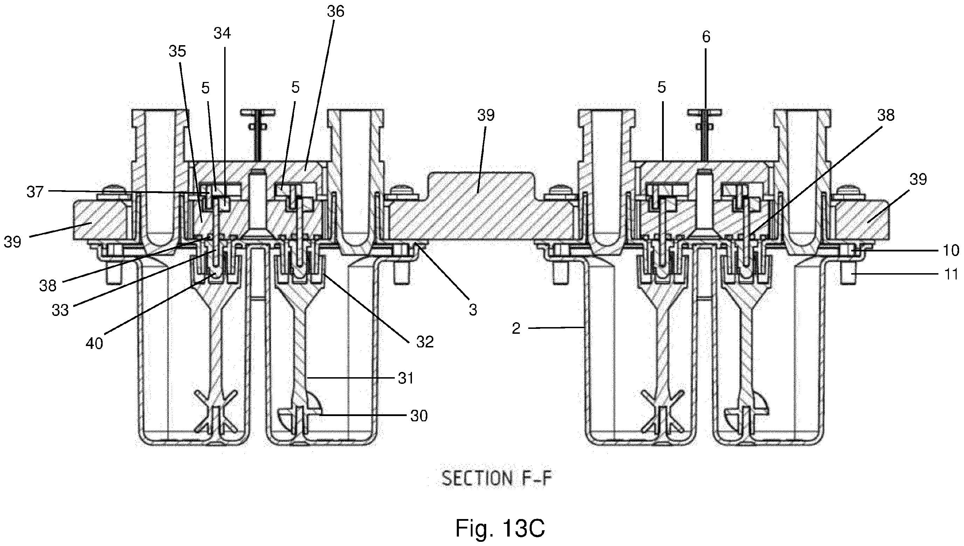

A drive mechanism (shown best in Figure 13C), in the form of a stirrer bar 5, is provided to rotate the impeller agitators 4 about the axes of their shafts 31. The stirrer bar 5 is held between stationary lower 35 and upper 36 clamp plates and is connected to each impeller by a respective pin wheel mechanism. More particularly, the stirrer bar has holes 7 at both ends which interface to an external drive linkage (not shown) for connecting to an external source of motive power of the bioreactor system. The linkage moves the stirrer bar with a circulating motion which induces the pin wheel mechanisms to rotate their respective impellers. A handle is connected directly to the lid 3 through aligned slots in the clamp plates and stirrer bar, the slot in the stirrer bar being sized so that handle does not interfere with the motion of the stirrer bar. Conveniently, the upper clamp plates 36 and the side plates 39 can be integrated as a single component. The stirrer bars 5, drive shafts 33, rotatable discs 34, lower clamp plates 35, upper clamp plates 36, pins 37, O-rings 38, side plates 39 and interfacing connectors 40 form a multi-use consumable part of the system which is suitable for repeat (although not indefinite) use, while other parts of the bioreactor system, such as the bioreaction vessels 2, lids 3, agitators 4, form a single-use consumable part of the system which is not intended for repeat use. In Figures 10 to 13, the multi-use consumable part is shown interfacing to two single-use consumable parts, but a multi-use consumable part such as this may be extended as needed to interface to any number of single-use consumable parts.

Each pin wheel mechanism comprises a pin 37 extending from the stirrer bar 5 into rotatable discs 34 situated in matching recesses formed in the lower clamp plate 35. When the stirrer bar is moved in a circulating motion, the rotatable discs are rotated by the pins. A drive shaft 33 connects each rotatable disc to its impeller via the respective impeller connector 32. Thus, the circulating motion of the stirrer bar, relative to the upper and lower stationary clamp plates, drives the rotatable discs, which in-turn rotates the drive shafts to turn the impellers. The drive shaft is connected to its respective impeller connector 32 by an interfacing connector 40.

O-rings 38 are provided around each drive shaft 33 in the lower clamp plate 35 to form an airtight seal around the drive shaft. This prevents off-gases from the bioreaction and gases which are maintained in the headspace between the bioreaction fluid and the lid 3 from escaping through the lid 3.

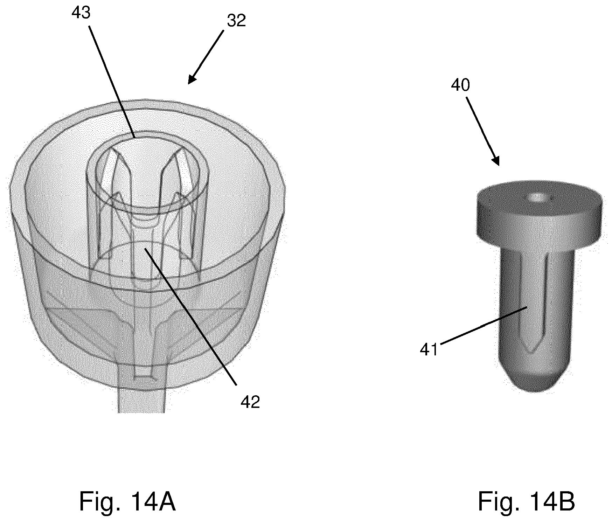

Figure 14A shows a perspective transparent view of an impeller connector 32, and Figure 14B shows a perspective view of an interfacing connector 40 at the end of the drive shaft 33 which connects the drive shaft to the impeller connector. The interfacing connector 40 inserts into a central receiving well 43 of the impeller connector 32. Side ridges 41 are provided on the interfacing connector which slide into corresponding slots 42 in the receiving well 43. Thus, when the interfacing connector 40 is rotated by the drive shaft 33, the impeller connector 32, and hence also the impeller 4, are rotated. The lower end of the interfacing connector 40 is tapered and leading edges of the side ridges are pointed to promote self-alignment of each interfacing connector 40 and its corresponding drive shaft 33 by its impeller connector 32 when the consumable is assembled. This self-aligning ability allows the consumable to be supplied to a user in two parts for assembly together by the user: one part comprising the vessels 2, impellers 4 and lid 3, and another part comprising the side plates 39 and drive mechanism (i.e. lower 35 and upper 36 clamp plates, stirrer bar 5, pin wheel mechanisms and drive shafts 33).

The features disclosed in the description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

## Claims

1. A consumable (1) for a bioreactor system, the consumable comprising:
a plurality of bioreaction vessels (2), each suitable for holding the contents of a bioreaction,
wherein the plurality of bioreaction vessels are provided as a single-piece construction, and each bioreaction vessel includes an agitator (4) configured to agitate the contents of the respective bioreaction vessel, each agitator having a stirring end which extends into the bioreaction vessel and a drive end.

2. The consumable of claim 1, wherein the consumable further comprises a drive mechanism (5) connected or connectable to the drive ends of the agitators of the plurality of bioreaction vessels and configured to move the agitators with identical synchronised motions.

3. The consumable of claim 2, wherein the drive mechanism includes an interface (7) to an external drive linkage which allows connection to an external source of motive power provided in the bioreactor system.

4. The consumable of claim 2 or 3, wherein the plurality of bioreaction vessels are arranged in one or more rows and the drive mechanism is a stirrer bar extending adjacent to and parallel with the one or more rows.

5. The consumable of any preceding claim wherein the stirring ends of the agitators are paddles.

6. The consumable of claim 5 wherein the agitators are connected to the drive mechanism at their drive ends via respective ball and socket joints (8), or via respective living hinges (23).

7. The consumable of claim 5 or 6 wherein the paddles are tapered such that the widths of the paddles decrease with increasing distance from their drive ends.

8. The consumable of any of claims 5 - 7 wherein each agitator has a pivot point (22) for its synchronised motion between its drive end and its stirring end, and each bioreaction vessel includes a flexible element (16) around the respective agitator at the pivot point, the flexible element providing a seal which closes off the bioreaction vessel at the agitator.

9. The consumable of claim 8 wherein the flexible element is made of a thermoplastic, elastomer or silicone.

10. The consumable of any of claims 1 - 4 wherein the stirring ends of the agitators are impellers.

11. The consumable of any preceding claim wherein the bioreaction vessels comprise respective outgassing ports (10) for exhausting gas from the bioreaction.

12. The consumable of claim 11 wherein each outgassing port includes a tortuous gas path (15) for controlled gas exhaust therethrough.

13. The consumable of any preceding claim wherein the plurality of the bioreaction vessels are rigid containers.

14. A bioreactor system comprising the consumable of any preceding claim and a source of motive power for moving the agitators of the consumable.

15. The bioreactor system of claim 14 further comprising a control unit for programmably controlling the movement of the agitators.
